# EUROPEAN PATENT APPLICATION

(11) **EP 4 180 536 A1**
(43) Date of publication of application: **17.05.2023**
(21) Application number: 21838010.3
(22) Date of filing: 09.07.2021
(51) Int. Cl.: C12Q 1/6813, C12N 15/11, C12Q 1/6883, G01N 33/50, G01N 33/53

(54) **METHOD FOR DETECTING SEVERITY OF ATOPIC DERMATITIS**

(30) Priority: 10.07.2020 JP 2020119040
(71) Applicant: Kao Corporation, Chuo-ku Tokyo 103-8210 (JP)
(72) Inventor: TAKADA, Naoto, Haga-gun, Tochigi 321-3497 (JP); KUWANO, Tetsuya, Haga-gun, Tochigi 321-3497 (JP); INOUE, Takayoshi, Haga-gun, Tochigi 321-3497 (JP)
(74) Representative: Vossius & Partner Patentanwälte Rechtsanwälte mbB
(86) International application number: PCT/JP2021/025988
(87) International publication number: WO 2022/009988

(57) **Abstract**

Provided are a marker for detecting the severity of atopic dermatitis, and a method for detecting the severity of atopic dermatitis using the marker. The method for detecting the severity of atopic dermatitis in a test subject comprises a step of measuring an expression level of at least one gene selected from the group of 9 genes consisting of CAPN1, NCOR2, PPP1R9B, PLP2, GRN, PPP1R12C, LOC146880, SLC31A1 and SYVN1 or an expression product thereof for a biological sample collected from the test subject.

## Description

### Field of the Invention

The present invention relates to a method for detecting the severity of atopic dermatitis using a marker for the severity of atopic dermatitis.

### Background of the Invention

Atopic dermatitis (hereinafter, also referred to as "AD") is eczematous skin disease which develops mainly in people having an atopic predisposition. Typical symptoms of atopic dermatitis are chronic and recurrent itchiness, eruption, erythema, and the like which occur bilaterally and symmetrically, as well as incomplete keratinization, decline in barrier function, dry skin, and the like. Most cases of atopic dermatitis occur in childhood, and children tend to outgrow atopic dermatitis. However, the number of adult or intractable atopic dermatitis cases has also increased in recent years.

The presence or absence of development or severity of atopic dermatitis can be evaluated to some extent by the visual observation of the skin or the image analysis of skin surface. Also, examination methods using TARC (thymus and activation-regulated chemokine) values or SCCA2 (squamous cell carcinoma antigen 2) values in blood as indexes have recently been used as methods which can objectively determine the severity of atopic dermatitis. These are used as indexes which sharply reflect the exacerbation of atopic dermatitis, and are used not only to evaluate the presence or absence of development or severity of atopic dermatitis but to educate patients or determine therapeutic strategies (see Non Patent Literatures 1, 2, and 3).

However, the examination methods using TARC values or SCCA2 values in blood as indexes require blood collection and are therefore invasive methods. Another problem thereof is to require time for obtaining results.

Meanwhile, techniques of examining the current and future *in vivo* physiological states of humans by the analysis of nucleic acids such as DNA or RNA in biological samples have been developed. The analysis using nucleic acids has the advantages that abundant information can be obtained by single analysis because exhaustive analysis methods have been established, and that analysis results are easy to connect functionally on the basis of many research reports on single-nucleotide polymorphism, RNA functions, and the like. Nucleic acids derived from a living body can be extracted from body fluids such as blood, secretions, tissues, and the like, it has recently been reported that: RNA contained in skin surface lipids (SSL) can be used as a sample for biological analysis; and marker genes of the epidermis, the sweat gland, the hair follicle and the sebaceous gland can be detected from SSL (Patent Literature 1). It has been further reported that marker genes for atopic dermatitis can be detected from SSL (Patent Literature 2) .
[Patent Literature 1] WO 2018/008319
[Patent Literature 2] JP-A-2020-74769

[Non Patent Literature 1] Sugawara et al., Allergy (2002) 57: 180-181
[Non Patent Literature 2] Ohta et al., Ann Clin Biochem. (2012) 49: 277-84
[Non Patent Literature 3] Kato et al., The Japanese Journal of Dermatology (2018) 128: 2431-2502

### Summary of the Invention

The present invention relates to the following 1) to 4) .
1) A method for detecting severity of atopic dermatitis in a test subject, comprising a step of measuring an expression level of at least one gene selected from the group of 9 genes consisting of CAPN1, NCOR2, PPP1R9B, PLP2, GRN, PPP1R12C, LOC146880, SLC31A1 and SYVN1 or an expression product thereof for a biological sample collected from the test subject.
2) Use of at least one gene selected from the group of 9 genes consisting of CAPN1, NCOR2, PPP1R9B, PLP2, GRN, PPP1R12C, LOC146880, SLC31A1 and SYVN1 or an expression product thereof derived from a biological sample collected from a test subject, as a marker for severity of atopic dermatitis.
3) A test kit for detecting severity of atopic dermatitis, the kit being used in a method according to 1), and comprising an oligonucleotide which specifically hybridizes to the gene, or an antibody which recognizes an expression product of the gene.
4) A marker for detecting severity of atopic dermatitis comprising at least one gene selected from the group of 9 genes consisting of CAPN1, NCOR2, PPP1R9B, PLP2, GRN, PPP1R12C, LOC146880, SLC31A1 and SYVN1 or an expression product thereof.

### Detailed Description of the Invention

The present invention relates a provision of a marker for detecting the severity of atopic dermatitis, and a method for detecting the severity of atopic dermatitis using the marker.

The present inventors collected SSL from atopic dermatitis patients differing in the severity and healthy persons and exhaustively analyzed the expressed state of RNA contained in the SSL as sequence information, and consequently found that the expression levels of particular genes significantly differ between the patients differing in the severity and between the patients and the healthy persons, and the severity of atopic dermatitis can be detected on the basis of this as an index.

The present invention enables the severity of atopic dermatitis to be conveniently and noninvasively detected early in an accurate and objective manner.

All patent literatures, non patent literatures, and other publications cited herein are incorporated herein by reference in their entirety.

In the present invention, the term "nucleic acid" or "polynucleotide" means DNA or RNA. The DNA includes all of cDNA, genomic DNA, and synthetic DNA. The "RNA" includes all of total RNA, mRNA, rRNA, tRNA, non-coding RNA and synthetic RNA.

In the present invention, the "gene" encompasses double-stranded DNA including human genomic DNA as well as single-stranded DNA (positive strand) including cDNA, single-stranded DNA having a sequence complementary to the positive strand (complementary strand), and their fragments, and means matter containing some biological information in sequence information on bases constituting DNA.

The "gene" encompasses not only a "gene" represented by a particular nucleotide sequence but a nucleic acid encoding a congener (i.e., a homolog or an ortholog), a variant such as gene polymorphism, and a derivative thereof.

The names of genes disclosed herein follow Official Symbol described in NCBI ([www.ncbi.nlm.nih.gov/]).

In the present invention, the "expression product" of a gene conceptually encompasses a transcription product and a translation product of the gene. The "transcription product" is RNA resulting from the transcription of the gene (DNA), and the "translation product" means a protein which is encoded by the gene and translationally synthesized on the basis of the RNA.

In the present invention, the "atopic dermatitis" refers to a disease which has eczema with itch in principal pathogen and repeats exacerbation and remission, and most of its patients reportedly have an atopic predisposition. Examples of the atopic predisposition include i) family history and/or previous medical history (any or a plurality of diseases among bronchial asthma, allergic rhinitis/ conjunctivitis, and atopic dermatitis), or ii) a predisposition to easily produce an IgE antibody.

In the present invention, the "severity of atopic dermatitis" means the degree of progression of atopic dermatitis and is classified into, for example, no symptoms, minor, mild (low grade), moderate (intermediate grade), and severe (high grade), depending on the state of eruption.

Examples of the nature of eruption include erythema, edema/oozing/papule (solid or serous), exudate/eschar, excoriation, lichenification, dryness, itch, prurigo nodule, scale (pityroid, foliate, membranous, etc.), blister, pustule, erosion, and ulcer. For example, Eczema Area and Severity Index ("EASI" <Exp Dermatol, 2001; 10: 11-18>), Investigator's Global Assessment ("IGA" <J Am Acad Dermatol, 2016; 74: 288-94>) as well as Atopic Dermatitis Severity Classification (The Japanese Journal of Dermatology <2001; 111: 2023-2033, The Japanese Journal of Dermatology, 1998; 108: 1491-1496>) provided by the Advisory Committee for Atopic Dermatitis Severity Classification of Japanese Dermatological Association and Severity coring of Atopic Dermatitis ("SCORAD" <Dermatology, 1993; 186: 23-31> are known as severity classification.

For example, EASI is a score from 0 to 72 which is calculated on the basis of scores based on four symptoms, erythema, edema/oozing/papule, excoriation, and lichenification, in each of the head and neck, the body trunk, the upper limbs, and the lower limbs as assessed sites, and the percentage (%) of areas with the four symptoms based on the whole assessed sites.

In IGA, a physician or a researcher serving as an evaluator comprehensively assesses the state of eruption for systemic or particular assessed sites and makes assessment on five scales "no symptom", "minor", "low grade", "intermediate grade" and "high grade". Six scales including "highest grade" may be used. The IGA scores can be used by conversion to ordinal scales such as "no symptom = 0", "minor = 1", "low grade = 2", "intermediate grade = 3", "high grade = 4" and "highest grade = 5".

In the detection of severity of atopic dermatitis of the present invention, "no symptom", "low grade", "intermediate grade" and "high grade" can be detected, and "no symptom", "low grade" and "intermediate grade" are preferably detected.

In the present invention, the "low grade" refers to a pathological condition composed mainly of dryness and low-grade erythema, scale, or the like and corresponds to an EASI score of larger than 0 and smaller than 6 or an IGA score of 2. The "intermediate grade" refers to a pathological condition composed mainly of erythema up to the intermediate grade, scale, a small number of papules, excoriation, or the like and corresponds to an EASI score of 6 or larger and smaller than 23 or an IGA score of 3. The "high grade" refers to a pathological condition composed mainly of erythema accompanied by high-grade puffiness/edema/oozing or lichenification, a rash of papule, high-grade scale, scabbing, vesicle, erosion, a large number of excoriation, prurigo nodule, or the like and corresponds to an EASI score of 23 or larger and 72 or smaller or an IGA score of 4. The "no symptom" refers to the same level as that in healthy persons without symptoms as a result of remission or almost remission.

In the assessment of "no symptom", "low grade", "intermediate grade" and "high grade" using an EASI score or an IGA score, the range of the score based on which each assessment is made is not limited to those described above and can be appropriately determined.

In the present invention, the term "detection" of severity of atopic dermatitis means to elucidate the severity of atopic dermatitis, and may be used interchangeably with the term "examination", "measurement", "determination", or "assistance of evaluation". In the present invention, the term "detection", "examination", "measurement", "determination" or "evaluation" does not include the diagnosis of severity of atopic dermatitis by a physician.

As shown in Examples mentioned later, RNA expression analysis was conducted by procedures 1) to 2) given below for 14 healthy subjects and a total of 29 atopic dermatitis patients including 18 patients diagnosed with mild (score: larger than 0 and smaller than 6) and 11 patients diagnosed with moderate (score: 6 or larger and smaller than 23) in accordance with the severity of EASI. As a result, the expression of 5 genes of CAPN1, GRN, NCOR2, PLP2, and PPP1R9B was increased in a severity-dependent manner.
1) Data (read count values) on the expression level of RNA extracted from SSL of 14 healthy subjects and 29 atopic dermatitis patients (adult) is obtained.
2) The read count values are converted to RPM values which normalize the read count values for difference in the total number of reads among samples. Values thereof plus integer 1 (RPM + 1 values) are converted to logarithmic values to base 2 (Log₂(RPM + 1) values), based on which a test was conducted by the Tukey method among 3 groups (healthy subjects, mild patients and moderate patients) to select genes which attain a p value of less than 0.1 in all of healthy subjects vs. mild patients, mild patients vs. moderate patients, and healthy subjects vs. moderate patients.

In this context, the "p value" refers to the probability of observing more extreme statics than statics actually calculated from data under null hypothesis in a statistical test. Thus, a smaller "p value" indicates more significant difference between subjects to be compared.

RNA expression analysis was conducted by the procedures 1) to 2) in the same manner as above for 14 healthy subjects and a total of 29 atopic dermatitis patients including 17 patients diagnosed with mild and 12 patients diagnosed with moderate by comprehensive assessment on 5 scales of 0 (no symptom), 1 (minor), 2 (mild), 3 (moderate), and 4 (severe) by IGA on a region of the face. As a result, the expression of 3 genes of LOC146880, PPP1R12C, and SYVN1 was increased in a severity-dependent manner while the expression of SLC31A1 was decreased in a severity-dependent manner.

Thus, a gene selected from the group of 9 genes consisting of CAPN1, NCOR2, PPP1R9B, PLP2, GRN, PPP1R12C, LOC146880, SLC31A1 and SYVN1 or an expression product thereof is capable of serving as a marker for detecting the severity of atopic dermatitis in order to detect the severity of atopic dermatitis.

These 9 genes are each capable of serving alone as a marker for detecting the severity of atopic dermatitis and may be used in combination of two or more thereof.

Preferably, the marker for detecting is selected from 5 genes of CAPN1, NCOR2, PPP1R9B, PLP2 and GRN among the 9 genes in order to detect the severity of atopic dermatitis based on EAST, and is selected from 4 genes of PPP1R12C, LOC146880, SLC31A1 and SYVN1 among the 9 genes in order to detect the severity of atopic dermatitis based on IGA.

These 9 genes are novel markers for determining the severity of atopic dermatitis whose relation to the severity of atopic dermatitis has not been reported so far.

The gene capable of serving as a marker for detecting the severity of atopic dermatitis (hereinafter, also referred to as a "target gene") also encompasses a gene having a nucleotide sequence substantially identical to the nucleotide sequence of DNA constituting the gene as long as the gene is capable of serving as a biomarker for detecting the severity of atopic dermatitis. In this context, the substantially identical nucleotide sequence means a nucleotide sequence having 90% or higher, preferably 95% or higher, more preferably 98% or higher, further more preferably 99% or higher identity to the nucleotide sequence of DNA constituting the gene, for example, when searched using homology calculation algorithm NCBI BLAST under conditions of expectation value = 10; gap accepted; filtering = ON; match score = 1; and mismatch score = -3.

The method for detecting the severity of atopic dermatitis according to the present invention includes a step of measuring an expression level of a target gene which is in one aspect at least one gene selected from the group of 9 genes consisting of CAPN1, NCOR2, PPP1R9B, PLP2, GRN, PPP1R12C, LOC146880, SLC31A1 and SYVN1 or an expression product thereof for a biological sample collected from a test subject.

In the method for detecting the severity of atopic dermatitis according to the present invention, the test subject is not limited by sex, age and race, etc. and can include infants to aged persons. Preferably, the test subject is a human who needs or desires the detection of severity of atopic dermatitis. The test subject is, for example, a human having atopic dermatitis, a human suspected of developing atopic dermatitis, or a human genetically having a predisposition to atopic dermatitis.

The biological sample used in the present invention can be a tissue or a biomaterial in which the expression of the gene of the present invention varies depending on the severity of atopic dermatitis. Examples thereof specifically include the organs, skin, blood, urine, saliva, sweat, stratum corneum, skin surface lipids (SSL), body fluids such as tissue exudates, serum, plasma and others prepared from blood, feces, and hair, and preferably include the skin, stratum corneum, and skin surface lipids (SSL), more preferably skin surface lipids (SSL). Examples of the site of the skin from which SSL is collected include, but are not particularly limited to, the skin at an arbitrary site of the body, such as head, face, neck, body trunk, and limbs. A site having high secretion of sebum, for example, the skin of head or face, is preferred, and the facial skin is more preferred. The site of the skin from which SSL is collected may be an eruption site having atopic dermatitis or a non-eruption site without atopic dermatitis, and is preferably an eruption site or a non-eruption site near an eruption site. In this context, the term "near an eruption site" refers to a range within 10 cm adjacent to the eruption site.

In this context, the "skin surface lipids (SSL)" refer to a lipid-soluble fraction present on skin surface, and is also called sebum. In general, SSL mainly contains secretions secreted from the exocrine gland such as the sebaceous gland in the skin, and is present on skin surface in the form of a thin layer that covers the skin surface. SSL contains RNA expressed in skin cells (see Patent Literature 1 described above). In the present specification, the "skin" is a generic name for regions containing tissues such as the stratum corneum, epidermis, dermis, and hair follicle as well as sweat gland, sebaceous gland and other glands, unless otherwise specified.

Any approach for use in the collection or removal of SSL from the skin can be adopted for the collection of SSL from the skin of a test subject. Preferably, an SSL-absorbent material or an SSL-adhesive material mentioned later, or a tool for scraping SSL from the skin can be used. The SSL-absorbent material or the SSL-adhesive material is not particularly limited as long as the material has affinity for SSL. Examples thereof include polypropylene and pulp. More detailed examples of the procedure of collecting SSL from the skin include a method of allowing SSL to be absorbed to a sheet-like material such as an oil blotting paper or an oil blotting film, a method of allowing SSL to adhere to a glass plate, a tape, or the like, and a method of collecting SSL by scraping with a spatula, a scraper, or the like. In order to improve the adsorbability of SSL, an SSL-absorbent material impregnated in advance with a solvent having high lipid solubility may be used. On the other hand, the SSL-absorbent material preferably has a low content of a solvent having high water solubility or water because the adsorption of SSL to a material containing the solvent having high water solubility or water is inhibited. The SSL-absorbent material is preferably used in a dry state.

The RNA-containing SSL collected from the test subject may be preserved for a given period. The collected SSL is preferably preserved under low-temperature conditions as rapidly as possible after collection in order to minimize the degradation of contained RNA. The temperature conditions for the preservation of RNA-containing SSL according to the present invention may be 0°C or lower and are preferably from -20 ± 20°C to -80 ± 20°C, more preferably from -20 ± 10°C to -80 ± 10°C, further more preferably from -20 ± 20°C to -40 ± 20°C, further more preferably from -20 ± 10°C to -40 ± 10°C, further more preferably -20 ± 10°C, further more preferably -20 ± 5°C. The period of preservation of the RNA-containing SSL under the low-temperature conditions is not particularly limited and is preferably 12 months or shorter, for example, 6 hours or longer and 12 months or shorter, more preferably 6 months or shorter, for example, 1 day or longer and 6 months or shorter, further more preferably 3 months or shorter, for example, 3 days or longer and 3 months or shorter.

In the present invention, examples of the measurement object for the expression level of a target gene or an expression product thereof include cDNA artificially synthesized from RNA, DNA encoding the RNA, a protein encoded by the RNA, a molecule which interacts with the protein, a molecule which interacts with the RNA, and a molecule which interacts with the DNA. In this context, examples of the molecule which interacts with the RNA, the DNA or the protein include DNA, RNA, proteins, polysaccharides, oligosaccharides, monosaccharides, lipids, fatty acids, and their phosphorylation products, alkylation products, and sugar adducts, and complexes of any of them. The expression level comprehensively means the expression level (expressed amount) or activity of the gene or the expression product.

In a preferred aspect, in the method of the present invention, SSL is used as a biological sample. In this case, in the method of the present invention, the expression level of RNA contained in SSL is analyzed. Specifically, RNA is converted to cDNA through reverse transcription, followed by the measurement of the cDNA or an amplification product thereof.

In the extraction of RNA from SSL, a method which is usually used in RNA extraction or purification from a biological sample, for example, phenol/chloroform method, AGPC (acid guanidinium thiocyanate-phenol-chloroform extraction) method, a method using a column such as TRIzol(R), RNeasy(R), or QIAzol(R), a method using special magnetic particles coated with silica, a method using magnetic particles for solid phase reversible immobilization, or extraction with a commercially available RNA extraction reagent such as ISOGEN can be used.

In the reverse transcription, primers which target particular RNA to be analyzed may be used, and random primers are preferably used for more comprehensive nucleic acid preservation and analysis. In the reverse transcription, common reverse transcriptase or reverse transcription reagent kit can be used. Highly accurate and efficient reverse transcriptase or reverse transcription reagent kit is suitably used. Examples thereof include M-MLV reverse transcriptase and its modified forms, and commercially available reverse transcriptase or reverse transcription reagent kits, for example, PrimeScript(R) Reverse Transcriptase series (Takara Bio Inc.) and SuperScript(R) Reverse Transcriptase series (Thermo Fisher Scientific, Inc.). SuperScript(R) III Reverse Transcriptase, SuperScript(R) VILO cDNA Synthesis kit (both from Thermo Fisher Scientific, Inc.), and the like are preferably used.

The temperature of extension reaction in the reverse transcription is adjusted to preferably 42°C ± 1°C, more preferably 42°C ± 0.5°C, further more preferably 42°C ± 0.25°C, while its reaction time is adjusted to preferably 60 minutes or longer, more preferably from 80 to 120 minutes.

In the case of using RNA, cDNA or DNA as a measurement object, the method for measuring the expression level can be selected from nucleic acid amplification methods typified by PCR using DNA primers which hybridize thereto, real-time RT-PCR, multiplex PCR, SmartAmp, and LAMP, hybridization using a nucleic acid probe which hybridizes thereto (DNA chip, DNA microarray, dot blot hybridization, slot blot hybridization, Northern blot hybridization, and the like), a method of determining a nucleotide sequence (sequencing), and combined methods thereof.

In PCR, one particular DNA to be analyzed may be amplified using a primer pair which targets the particular DNA, or a plurality of particular DNAs may be amplified at the same time using a plurality of primer pairs. Preferably, the PCR is multiplex PCR. The multiplex PCR is a method of amplifying a plurality of gene regions at the same time by using a plurality of primer pairs at the same time in a PCR reaction system. The multiplex PCR can be carried out using a commercially available kit (e.g., Ion AmpliSeq Transcriptome Human Gene Expression Kit; Life Technologies Japan Ltd.).

The temperature of annealing and extension reaction in the PCR depends on the primers used and therefore cannot be generalized. In the case of using the multiplex PCR kit described above, the temperature is preferably 62°C ± 1°C, more preferably 62°C ± 0.5°C, further more preferably 62°C ± 0.25°C. Thus, preferably, the annealing and the extension reaction are performed by one step in the PCR. The time of the step of the annealing and the extension reaction can be adjusted depending on the size of DNA to be amplified, and the like, and is preferably from 14 to 18 minutes. Conditions for denaturation reaction in the PCR can be adjusted depending on the size of DNA to be amplified, and are preferably from 95 to 99°C and from 10 to 60 seconds. The reverse transcription and the PCR using the temperatures and the times as described above can be carried out using a thermal cycler which is generally used for PCR.

The reaction product obtained by the PCR is preferably purified by the size separation of the reaction product. By the size separation, the PCR reaction product of interest can be separated from the primers and other impurities contained in the PCR reaction solution. The size separation of DNA can be performed using, for example, a size separation column, a size separation chip, or magnetic beads which can be used in size separation. Preferred examples of the magnetic beads which can be used in size separation include magnetic beads for solid phase reversible immobilization (SPRI) such as Ampure XP.

The purified PCR reaction product may be subjected to further treatment necessary for conducting subsequent quantitative analysis. For example, for DNA sequencing, the purified PCR reaction product may be prepared into an appropriate buffer solution, the PCR primer regions contained in DNA amplified by PCR may be cleaved, and an adaptor sequence may be further added to the amplified DNA. For example, the purified PCR reaction product can be prepared into a buffer solution, and the removal of the PCR primer sequences and adaptor ligation can be performed for the amplified DNA. If necessary, the obtained reaction product can be amplified to prepare a library for quantitative analysis. These operations can be performed, for example, using 5 × VILO RT Reaction Mix attached to SuperScript(R) VILO cDNA Synthesis kit (Life Technologies Japan Ltd.), 5 × Ion AmpliSeq HiFi Mix attached to Ion AmpliSeq Transcriptome Human Gene Expression Kit (Life Technologies Japan Ltd.), and Ion AmpliSeq Transcriptome Human Gene Expression Core Panel according to a protocol attached to each kit.

In the case of measuring the expression level of a target gene or a nucleic acid derived therefrom by use of Northern blot hybridization, for example, probe DNA is first labeled with a radioisotope, a fluorescent material, or the like. Subsequently, the obtained labeled DNA is allowed to hybridize to biological sample-derived RNA transferred to a nylon membrane or the like in accordance with a routine method. Then, the formed duplex of the labeled DNA and the RNA is measured by detecting a signal derived from the label.

In the case of measuring the expression level of a target gene or a nucleic acid derived therefrom by use of RT-PCR, for example, cDNA is first prepared from biological sample-derived RNA in accordance with a routine method. This cDNA is used as a template, and a pair of primers (a positive strand which binds to the cDNA (- strand) and an opposite strand which binds to a + strand) prepared so as to be able to amplify the target gene of the present invention is allowed to hybridize thereto. Then, PCR is performed in accordance with a routine method, and the obtained amplified double-stranded DNA is detected. In the detection of the amplified double-stranded DNA, for example, a method of detecting labeled double-stranded DNA produced by the PCR using primers labeled in advance with RI, a fluorescent material, or the like can be used.

In the case of measuring the expression level of a target gene or a nucleic acid derived therefrom by use of a DNA microarray, for example, an array in which at least one nucleic acid (cDNA or DNA) derived from the target gene of the present invention is immobilized on a support is used. Labeled cDNA or cRNA prepared from mRNA is allowed to bind onto the microarray, and the expression level of the mRNA can be measured by detecting the label on the microarray.

The nucleic acid to be immobilized in the array can be a nucleic acid which specifically hybridizes (i.e., substantially only to the nucleic acid of interest) under stringent conditions, and may be, for example, a nucleic acid having the whole sequence of the target gene of the present invention or may be a nucleic acid consisting of a partial sequence thereof. In this context, examples of the "partial sequence" include nucleic acids consisting of at least 15 to 25 bases. In this context, examples of the stringent conditions can usually include washing conditions on the order of "1 × SSC, 0.1% SDS, and 37°C". Examples of the more stringent hybridization conditions can include conditions on the order of "0.5 × SSC, 0.1% SDS, and 42°C". Examples of the much more stringent hybridization conditions can include conditions on the order of "0.1 × SSC, 0.1% SDS, and 65°C". The hybridization conditions are described in, for example, J. Sambrook et al., Molecular Cloning: A Laboratory Manual, Third Edition, Cold Spring Harbor Laboratory Press (2001).

In the case of measuring the expression level of a target gene or a nucleic acid derived therefrom by sequencing, examples thereof include analysis using a next-generation sequencer (e.g., Ion S5/XL system, Life Technologies Japan Ltd.). RNA expression can be quantified on the basis of the number of reads (read count) prepared by the sequencing.

The probe or the primers for use in the measurement described above, which correspond to the primers for specifically recognizing and amplifying the target gene of the present invention or a nucleic acid derived therefrom, or the probe for specifically detecting the RNA or the nucleic acid derived therefrom, can be designed on the basis of a nucleotide sequence constituting the target gene. In this context, the phrase "specifically recognize" means that a detected product or an amplification product can be confirmed to be the gene or the nucleic acid derived therefrom in such a way that, for example, substantially only the target gene of the present invention or the nucleic acid derived therefrom can be detected in Northern blot, or, for example, substantially only the nucleic acid is amplified in RT-PCR.

Specifically, an oligonucleotide containing a given number of nucleotides complementary to DNA consisting of a nucleotide sequence constituting the target gene of the present invention, or a complementary strand thereof can be used. In this context, the "complementary strand" refers to one strand of double-stranded DNA consisting of A:T (U for RNA) and/or G:C base pairs with respect to the other strand. The term "complementary" is not limited to the case of being a completely complementary sequence in a region with the given number of consecutive nucleotides, and can have preferably 80% or higher, more preferably 90% or higher, further more preferably 95% or higher, even more preferably 98% or higher identity of the nucleotide sequence. The identity of the nucleotide sequence can be determined by algorithm such as BLAST described above.

For use as a primer, the oligonucleotide can achieve specific annealing and strand extension. Examples thereof usually include oligonucleotides having a strand length of 10 or more bases, preferably 15 or more bases, more preferably 20 or more bases, and 100 or less bases, preferably 50 or less bases, more preferably 35 or less bases. For use as a probe, the oligonucleotide may achieve specific hybridization. An oligonucleotide can be used which has at least a portion or the whole of the sequence of DNA (or a complementary strand thereof) consisting of a nucleotide sequence constituting the target gene of the present invention, and has a strand length of, for example, 10 or more bases, preferably 15 or more bases, and, for example, 100 or less bases, preferably 50 or less bases, more preferably 25 or less bases.

In this context, the "oligonucleotide" can be DNA or RNA and may be synthetic or natural. The probe for use in hybridization is usually labeled for use.

In the case of measuring a translation product (protein) of the target gene of the present invention, a molecule which interacts with the protein, a molecule which interacts with the RNA, or a molecule which interacts with the DNA, a method such as protein chip analysis, immunoassay (e.g., ELISA), mass spectrometry (e.g., LC-MS/MS and MALDI-TOF/MS), one-hybrid method (PNAS 100, 12271-12276 (2003)), or two-hybrid method (Biol. Reprod. 58, 302-311 (1998)) can be used and can be appropriately selected depending on the measurement object.

For example, in the case of using the protein as a measurement object, the measurement is carried out by contacting an antibody against the expression product of the present invention with a biological sample, detecting a polypeptide in the sample bound with the antibody, and measuring the level thereof. For example, according to Western blot, the antibody described above is used as a primary antibody, and an antibody which binds to the primary antibody and which is labeled with, for example, a radioisotope, a fluorescent material or an enzyme is used as a secondary antibody so that the primary antibody is labeled, followed by the measurement of a signal derived from such a labeling material using a radiation meter, a fluorescence detector, or the like.

The antibody against the translation product may be a polyclonal antibody or a monoclonal antibody. These antibodies can be produced in accordance with a method known in the art. Specifically, the polyclonal antibody may be produced by using a protein which has been expressed in *E. coli* or the like and purified in accordance with a routine method, or synthesizing a partial polypeptide of the protein in accordance with a routine method, and immunizing a nonhuman animal such as a house rabbit therewith, followed by obtainment from the serum of the immunized animal in accordance with a routine method.

On the other hand, the monoclonal antibody can be obtained from hybridoma cells prepared by immunizing a nonhuman animal such as a mouse with a protein which has been expressed in *E. coli* or the like and purified in accordance with a routine method, or a partial polypeptide of the protein, and fusing the obtained spleen cells with myeloma cells. Alternatively, the monoclonal antibody may be prepared by use of phage display (Griffiths, A.D.; Duncan, A.R., Current Opinion in Biotechnology, Volume 9, Number 1, February 1998, pp. 102-108 (7)).

In this way, the expression level of the target gene of the present invention or the expression product thereof in a biological sample collected from a test subject is measured, and the severity of atopic dermatitis is detected on the basis of the expression level. The detection is specifically performed by comparing the measured expression level of the target gene of the present invention or the expression product thereof with a control level.

In the case of analyzing expression levels of a plurality of target genes by sequencing, as described above, read count values which are data on expression levels, RPM values which normalize the read count values for difference in the total number of reads among samples, values obtained by the conversion of the RPM values to logarithmic values to base 2 (Log₂RPM values) or logarithmic values to base 2 plus integer 1 (Log₂(RPM + 1) values), or normalized count values obtained using DESeq2 (Love MI et al. Genome Biol. 2014) or logarithmic values to base 2 plus integer 1 (Log₂(count + 1) values) are preferably used as an index. Also, values calculated by, for example, fragments per kilobase of exon per million reads mapped (FPKM), reads per kilobase of exon per million reads mapped (RPKM), or transcripts per million (TPM) which are general quantitative values of RNA-seq may be used. Further, signal values obtained by microarray method or corrected values thereof may be used. In the case of analyzing only a particular target gene by RT-PCR or the like, an analysis method of converting the expression level of the target gene to a relative expression level based on the expression level of a housekeeping gene, or an analysis method of quantifying an absolute copy number using a plasmid containing a region of the target gene (absolute quantification) is preferred. A copy number obtained by digital PCR may be used.

In this context, examples of the "control level" include an expression level of the target gene or the expression product thereof in a healthy subject when detecting a mild patient or in a mild patient when detecting a moderate patient. The expression level of the healthy person or the mild patient may be a statistic (e.g., a mean) of the expression level of the gene or the expression product thereof measured from a population of healthy persons or mild patients. When using a plurality of target genes, it is preferred to determine a standard expression level for each individual gene or expression product thereof.

The detection of severity of atopic dermatitis according to the present invention may be performed through an increase and/or decrease in the expression level of the target gene of the present invention or the expression product thereof. In this case, the expression level of the target gene or the expression product thereof in a biological sample derived from a test subject is compared with a cutoff value (reference value) of the gene or the expression product thereof. The cutoff value may be appropriately determined on the basis of a statistical numeric value, such as a mean or standard deviation, of the expression level based on standard data obtained in advance on the expression level of this target gene or expression product thereof in a healthy subject, mild patient, a moderate patient and a severe patient.

A discriminant (prediction model) which discriminates between a patient group having distinct severity (e.g., mild patients, moderate patients, or severe patients) and a healthy person (no symptom) is constructed using measurement values of an expression level of the target gene or the expression product thereof derived from an atopic dermatitis patient having distinct severity and an expression level of the target gene or the expression product thereof derived from a healthy person, and the severity of atopic dermatitis can be detected through the use of the discriminant. Specifically, a discriminant (prediction model) which discriminates between each patient group having distinct severity (e.g., mild patients, moderate patients, and severe patients) and a healthy person (no symptom) is constructed by using measurement values of an expression level of the target gene or the expression product thereof derived from an atopic dermatitis patient group having distinct severity and an expression level of the target gene or the expression product thereof derived from a healthy subject as teacher samples, and a cutoff value (reference value) which discriminates between the patient groups having distinct severity is determined on the basis of the discriminant. In the preparation of the discriminant, dimensional compression is performed by principal component analysis (PCA), and a principal component can be used as an explanatory variable.

The severity of atopic dermatitis in a test subject can be detected by similarly measuring a level of the target gene or the expression product thereof from a biological sample collected from the test subject, substituting the obtained measurement value into the discriminant, and comparing the results obtained from the discriminant with the reference value.

Algorithm known in the art such as algorithm for use in machine learning can be used as the algorithm in the construction of the discriminant. Examples of the machine learning algorithm include random forest, linear kernel support vector machine (SVM linear), rbf kernel support vector machine (SVM rbf), neural net, generalized linear model, regularized linear discriminant analysis, and regularized logistic regression. A prediction value is calculated by inputting data for the verification of the constructed prediction model, and a model which attains the prediction value most compatible with an actually measured value, for example, a model which attains the largest accuracy, can be selected as the optimum prediction model. Further, recall, precision, and an F value which is a harmonic mean thereof are calculated from a prediction value and an actually measured value, and a model having the largest F value can be selected as the optimum prediction model.

A method for determining the cutoff value (reference value) is not particularly limited, and the value can be determined in accordance with an approach known in the art. The value can be determined from, for example, an ROC (receiver operating characteristic) curve prepared using the discriminant. In the ROC curve, the probability (%) of producing positive results in positive patients (sensitivity) is plotted on the ordinate against a value (false positive fraction) of 1 minus the probability (%) of producing negative results in negative patients (specificity) on the abscissa. As for "true positive (sensitivity)" and "false positive (1 - specificity)" shown in the ROC curve, a value at which "true positive (sensitivity)" - "false positive (1 - specificity)" is maximized (Youden index) can be used as the cutoff value (reference value).

The test kit for detecting the severity of atopic dermatitis according to the present invention contains a test reagent for measuring an expression level of the target gene of the present invention or an expression product thereof in a biological sample separated from a patient. Specific examples thereof include a reagent for nucleic acid amplification and hybridization containing an oligonucleotide (e.g., a primer for PCR) which specifically binds (hybridizes) to the target gene of the present invention or a nucleic acid derived therefrom, and a reagent for immunoassay containing an antibody which recognizes an expression product (protein) of the target gene of the present invention. The oligonucleotide, the antibody, or the like contained in the kit can be obtained by a method known in the art as mentioned above.

The test kit can contain, in addition to the antibody or the nucleic acid, a labeling reagent, a buffer solution, a chromogenic substrate, a secondary antibody, a blocking agent, an instrument necessary for a test, a control reagent for use as a positive control or a negative control, a tool for collecting a biological sample (e.g., an oil blotting film for collecting SSL), and the like.

In relation to the embodiments mentioned above, the present invention further discloses the following aspects.
<1> A method for detecting severity of atopic dermatitis in a test subject, comprising a step of measuring an expression level of at least one gene selected from the group of 9 genes consisting of CAPN1, NCOR2, PPP1R9B, PLP2, GRN, PPP1R12C, LOC146880, SLC31A1 and SYVN1 or an expression product thereof for a biological sample collected from the test subject.
<2> The method according to <1>, wherein the severity is severity based on an EASI score or an IGA score.
<3> The method according to <2>, wherein the severity is severity based on the EASI score, and the measurement of the expression level is measurement of an expression level of at least one gene selected from the group of 5 genes consisting of CAPN1, NCOR2, PPP1R9B, PLP2 and GRN or an expression product thereof.
<4> The method according to <2>, wherein the severity is severity based on the IGA score, and the measurement of the expression level is measurement of an expression level of at least one gene selected from the group of 4 genes consisting of PPP1R12C, LOC146880, SLC31A1 and SYVN1 or an expression product thereof.
<5> The method according to any of <1> to <4>, wherein the severity is at least one member selected from the group consisting of no symptom, mild and moderate.
<6> The method according to any of <1> to <5>, wherein the expression level of the gene or the expression product thereof is measured as an expression level of mRNA.
<7> The method according to any of <1> to <6>, wherein the gene or the expression product thereof is RNA contained in skin surface lipids of the test subject.
<8> The method according to any of <1> to <7>, wherein the severity of atopic dermatitis is detected by comparing a measurement value of the expression level with a reference value of the gene or the expression product thereof.
<9> Use of at least one gene selected from the group of 9 genes consisting of CAPN1, NCOR2, PPP1R9B, PLP2, GRN, PPP1R12C, LOC146880, SLC31A1 and SYVN1 or an expression product thereof derived from a biological sample collected from a test subject, as a marker for severity of atopic dermatitis.
<10> The use according to <9>, wherein at least one gene selected from the group of 5 genes consisting of CAPN1, NCOR2, PPP1R9B, PLP2 and GRN or an expression product thereof is used, and the severity is severity based on an EASI score.
<11> The use according to <9>, wherein at least one gene selected from the group of 4 genes consisting of PPP1R12C, LOC146880, SLC31A1 and SYVN1 or an expression product thereof is used, and the severity is severity based on an IGA score.
<12> The use according to any of <9> to <11>, wherein the gene or the expression product thereof is mRNA contained in skin surface lipids collected from the test subject.
<13> A test kit for detecting severity of atopic dermatitis, the kit being used in the method according to any of <1> to <8>, and comprising an oligonucleotide which specifically hybridizes to the gene or a nucleic acid derived therefrom, or an antibody which recognizes an expression product of the gene.
<14> A marker for detecting severity of atopic dermatitis comprising at least one gene selected from the group of 9 genes consisting of CAPN1, NCOR2, PPP1R9B, PLP2, GRN, PPP1R12C, LOC146880, SLC31A1 and SYVN1 or an expression product thereof.

### Examples

Hereinafter, the present invention will be described in more detail with reference to Examples. However, the present invention is not limited to these examples.

### Example 1 Detection of severity of atopic dermatitis using RNA extracted from SSL

### 1) Diagnosis of atopic dermatitis patient and SSL collection

14 healthy subjects (from 25 to 57 years old, male) and 29 adults having atopic dermatitis (AD) (from 23 to 56 years old, male) were selected as test subjects. The atopic dermatitis patients were each diagnosed as having mild or moderate atopic dermatitis in terms of severity by a dermatologist. An EASI score (Hanifin et al., Exp dermatol. 10, 2001) was used in diagnosis. In accordance with the literature, patients having a score of larger than 0 and smaller than 6 were regarded as being mild, and patients having a score of 6 or larger and smaller than 23 were regarded as being moderate (Chopra et al., Br J Dermatol. 177, 2017). As a result, 18 patients were mild, and 11 patients were moderate.

Further, a region of the face of the atopic dermatitis patients described above was comprehensively assessed on 5 scales of 0 (no symptom), 1 (very minor), 2 (mild), 3 (moderate), and 4 (severe) using an IGA score. As a result, 17 patients were mild with a score of 2, and 12 patients were moderate with a score of 3. Sebum was collected from the whole face of each test subject using an oil blotting film. Then, the oil blotting film was transferred to a vial and preserved at -80°C for approximately 1 month until use in RNA extraction.

### 2) RNA preparation and sequencing

The oil blotting film of the above section 1) was cut into an appropriate size, and RNA was extracted using QIAzol Lysis Reagent (Qiagen N.V.) in accordance with the attached protocol. On the basis of the extracted RNA, cDNA was synthesized through reverse transcription at 42°C for 90 minutes using Superscript VILO cDNA Synthesis kit (Life Technologies Japan Ltd.). The primers used for reverse transcription reaction were random primers attached to the kit. A DNA library derived from 20802 genes was prepared by multiplex PCR from the obtained cDNA. The multiplex PCR was performed using Ion AmpliSeq Transcriptome Human Gene Expression Kit (Life Technologies Japan Ltd.) under conditions of [99°C, 2 min → (99°C, 15 sec -> 62°C, 16 min) × 20 cycles -> 4°C, hold]. The obtained PCR product was purified with Ampure XP (Beckman Coulter Inc.), followed by buffer reconstitution, primer sequence digestion, adaptor ligation, purification, and amplification to prepare a library. The prepared library was loaded on Ion 540 Chip and sequenced using Ion S5/XL system (Life Technologies Japan Ltd.).

### 3) Data analysis 1: Differential gene depending on severity classification based on EASI score

### i) Data used

Data (read count values) on the expression level of RNA derived from the test subjects measured in the above section 2) was converted to RPM values which normalized the read count values for difference in the total number of reads among samples. However, only 7429 genes which produced expression level data without missing values in 90% or more samples among all the samples were used in analysis given below. Logarithmic values to base 2 plus integer 1 (Log₂(RPM + 1) values) were used in order to approximate the RPM values, which followed negative binominal distribution, to normal distribution. Next, the samples were divided into 3 groups of healthy subjects, mild patients, and moderate patients in accordance with the severity classification based on EASI scores used in the assessment in the above section 1).

### ii) RNA expression analysis

A test was conducted by the Tukey method among the 3 groups on the basis of the SSL-derived RNA expression levels (Log₂(RPM + 1) values) of the healthy subjects, the mild patients, and the moderate patients measured in the above section i) to select genes which attained a p value of less than 0.1 in all of healthy subjects vs. mild patients, mild patients vs. moderate patients, and healthy subjects vs. moderate patients. As a result, the expression of 5 genes of CAPN1, GRNNCOR2, PLP2, and PPP1R9B was increased in a severity-dependent manner (Table 1).

**[Table 1-1]**

| | Mean | | | | | Standard deviation | | |
|---|---|---|---|---|---|---|---|---|
| | Healthy | Mild | Moderate | | | Healthy | Mild | Moderate |
| CAPN1 | 5.259 | 5.831 | 6.408 | | CAPN1 | 0.530 | 0.419 | 0.513 |
| GRN | 8.265 | 8.711 | 9.241 | | GRN | 0.423 | 0.656 | 0.357 |
| NCOR2 | 5.683 | 6.439 | 7.087 | | NCOR2 | 0.979 | 0.562 | 0.622 |
| PLP2 | 5.856 | 6.494 | 7.123 | | PLP2 | 0.663 | 0.793 | 0.779 |
| PPP1R9B | 7.143 | 7.991 | 8.459 | | PPP1R9B | 0.766 | 0.440 | 0.479 |

**[Table 1-2]**

| | p value (Tukey method) | | |
|---|---|---|---|
| | Healthy vs. Mild | Healthy vs. Moderate | Mild vs. Moderate |
| CAPN1 | 0.005 | Less than 0.001 | 0.009 |
| GRN | 0.054 | Less than 0.001 | 0.030 |
| NCOR2 | 0.017 | Less than 0.001 | 0.067 |
| PLP2 | 0.055 | Less than 0.001 | 0.085 |
| PPP1R9B | 0.001 | Less than 0.001 | 0.098 |

### 4) Data analysis 2: Differential gene depending on severity classification by comprehensive assessment (IGA score) on region of face i) Data used

Data (read count values) on the expression level of RNA derived from the test subjects measured in the above section 2) was converted to RPM values which normalized the read count values for difference in the total number of reads among samples. However, only 7429 genes which produced expression level data without missing values in 90% or more samples among all the samples were used in analysis given below. Logarithmic values to base 2 plus integer 1 (Log₂(RPM + 1) values) were used in order to approximate the RPM values, which followed negative binominal distribution, to normal distribution. Next, the samples were divided into 3 groups of healthy subjects, mild patients, and moderate patients in accordance with the severity classification based on comprehensive assessment (IGA scores) on a region of the face used in the assessment in the above section 1).

### ii) RNA expression analysis

A test was conducted by the Tukey method among the 3 groups on the basis of the SSL-derived RNA expression levels (Log₂(RPM + 1) values) of the healthy subjects, the mild patients, and the moderate patients measured in the above section i) to select genes which attained a p value of less than 0.1 in all of healthy subjects vs. mild patients, mild patients vs. moderate patients, and healthy subjects vs. moderate patients. As a result, the expression of 3 genes of LOC146880, PPP1R12C, and SYVN1 was increased in a severity-dependent manner while the expression of SLC31A1 was decreased in a severity-dependent manner (Table 2).

**[Table 2-1]**

| | Mean | | | | | Standard deviation | | |
|---|---|---|---|---|---|---|---|---|
| | Healthy | Mild | Moderate | | | Healthy | Mild | Moderate |
| LOC146880 | 5.549 | 6.118 | 6.727 | | LOC146880 | 0.724 | 0.774 | 0.629 |
| PPP1R12C | 5.566 | 6.308 | 6.930 | | PPP1R12C | 0.883 | 0.738 | 0.428 |
| SYVN1 | 4.032 | 4.449 | 4.982 | | SYVN1 | 0.579 | 0.545 | 0.371 |
| SLC31A1 | 8.702 | 7.922 | 7.054 | | SLC31A1 | 1.148 | 0.945 | 0.880 |

**[Table 2-2]**

| | p value (Tukey method) | | |
|---|---|---|---|
| | Healthy vs. Mild | Healthy vs. Moderate | Mild vs. Moderate |
| LOC146880 | 0.086 | Less than 0.001 | 0.076 |
| PPP1R12C | 0.019 | Less than 0.001 | 0.070 |
| SYVN1 | 0.077 | Less than 0.001 | 0.024 |
| SLC31A1 | 0.090 | Less than 0.001 | 0.067 |

## Claims

1. A method for detecting severity of atopic dermatitis in a test subject, comprising a step of measuring an expression level of at least one gene selected from the group of 9 genes consisting of CAPN1, NCOR2, PPP1R9B, PLP2, GRN, PPP1R12C, LOC146880, SLC31A1 and SYVN1 or an expression product thereof for a biological sample collected from the test subject.

2. The method according to claim 1, wherein the severity is severity based on an EASI (Eczema Area and Severity Index) score or an IGA (Investigator's Global Assessment) score.

3. The method according to claim 2, wherein the severity is severity based on the EASI score, and the measurement of the expression level is measurement of an expression level of at least one gene selected from the group of 5 genes consisting of CAPN1, NCOR2, PPP1R9B, PLP2 and GRN or an expression product thereof.

4. The method according to claim 2, wherein the severity is severity based on the IGA score, and the measurement of the expression level is measurement of an expression level of at least one gene selected from the group of 4 genes consisting of PPP1R12C, LOC146880, SLC31A1 and SYVN1 or an expression product thereof.

5. The method according to any one of claims 1 to 4, wherein the severity is at least one member selected from the group consisting of no symptom, mild and moderate.

6. The method according to any one of claims 1 to 5, wherein the expression level of the gene or the expression product thereof is measured as an expression level of mRNA.

7. The method according to any one of claims 1 to 6, wherein the gene or the expression product thereof is RNA contained in skin surface lipids of the test subject.

8. The method according to any one of claims 1 to 7, wherein the severity of atopic dermatitis is detected by comparing a measurement value of the expression level with a reference value of the gene or the expression product thereof.

9. Use of at least one gene selected from the group of 9 genes consisting of CAPN1, NCOR2, PPP1R9B, PLP2, GRN, PPP1R12C, LOC146880, SLC31A1 and SYVN1 or an expression product thereof derived from a biological sample collected from a test subject, as a marker for severity of atopic dermatitis.

10. The use according to claim 9, wherein the gene or the expression product thereof is mRNA contained in skin surface lipids collected from the test subject.

11. A test kit for detecting severity of atopic dermatitis, the kit being used in the method according to any one of claims 1 to 8, and comprising an oligonucleotide which specifically hybridizes to the gene or a nucleic acid derived therefrom, or an antibody which recognizes an expression product of the gene.

12. A marker for detecting severity of atopic dermatitis, comprising at least one gene selected from the group of 9 genes consisting of CAPN1, NCOR2, PPP1R9B, PLP2, GRN, PPP1R12C, LOC146880, SLC31A1 and SYVN1 or an expression product thereof.
